# EUROPÄISCHE PATENTSCHRIFT

(11) **EP 1 212 072 B1**
(45) Veröffentlichungstag und Bekanntmachung des Hinweises auf die Patenterteilung: **17.03.2004**
(21) Anmeldenummer: 00967663.6
(22) Anmeldetag: 12.09.2000
(51) Int. Cl.: A61K 35/78, A61P 31/00, A61P 11/00

(54) **PHARMAZEUTISCHE ZUSAMMENSETZUNG, UMFASSEND EUKALYPTUS- UND ORANGENÖL**
PHARMACEUTICAL COMPOSITION THAT CONTAINS EUCALYPTUS AND ORANGE OIL
COMPOSITION PHARMACEUTIQUE COMPRENANT DE L'HUILE D'EUCALYPTUS ET D'ORANGE

(30) Priorität: 16.09.1999 DE 29916014 U
(43) Veröffentlichungstag der Anmeldung: 12.06.2002
(73) Patentinhaber: AKH Arzneimittelkontor GmbH, 25524 Bekmünde (DE)
(72) Erfinder: UECK, Henning, 25524 Bekmünde (DE)
(74) Vertreter: Hamm, Volker, Dr.
(86) Internationale Anmeldenummer: PCT/EP2000/008903
(87) Internationale Veröffentlichungsnummer: WO 2001/019382

(56) Entgegenhaltungen:
- CH-A- 688 787
- DATABASE WPI Section Ch, Week 199612 Derwent Publications Ltd., London, GB; Class B04, AN 1996-114540 XP002159322 & RU 2 038 092 C (DUBINSKII R A), 27. Juni 1995 (1995-06-27)

## Beschreibung

Die Erfindung betrifft eine pharmazeutische Zusammensetzung für die orale Verabreichung in Form einer Hart- oder Weichgelatinekapsel oder einer Flüssigkeit umfassend Eukalyptusund Orangenöl in kombinierter Form, die vorzugsweise zur Behandlung von Erkrankungen des Respirationstraktes verwendet werden kann, die durch Mikroorganismen hervorgerufen werden.

Eine unkomplizierte Bronchitis ist definiert als entzündliche Erkrankung der unteren Atemwege und stellt eine der in der ärztlichen Praxis am häufigsten diagnostizierten Erkrankungen dar. Unterkühlung und Durchnässung in der kalten Jahreszeit sind oftmals die Wegbereiter der Bronchitis. Die Übertragung von Mensch zu Mensch erfolgt durch Tröpfcheninfektion, entsprechend kommt es häufig zum Auftreten von Epidemien. Als Auslöser kommen in erster Linie eine Reihe von Viren in Betracht, wie z.B. Influenzae-Viren, Parainfluenzae-Viren, Rhino-Viren, REO-Viren; Coxsackie-Viren, ECHO-Viren und Adeno-Viren. Eine primär bakterielle Genese ist sehr selten. Jedoch können virale Infekte eine bakterielle Superinfektion mit Haemophilus influenzae, Streptococcus pneumoniae seltener auch mit Staphylokokken nach sich ziehen.

Zu-Beginn der Erkrankung ist die Schleimhaut der zentralen Atemwege gerötet und ödematös geschwollen. Die Schleimhaut ist von polymorphkernigen Granulozyten und Lymphozyten infiltriert. Im Bronchiallumen findet man vermehrt muko-seröses Sekret. Nach Einwandern der Granulozyten in den Schleim wandelt sich dieser eitrig um: Im weiteren Verlauf gehen große Areale des bronchialen Flimmerepithels zugrunde und werden in das Bronchiallumen abgestoßen. Dieses Stadium der ulzerativen Bronchitis ist prädisponiert zur bakteriellen Superinfektion.

Als klinische Symptome der akuten Bronchitis treten zunächst über einige Tage ein trockener Husten verbunden mit Schmerzen hinter dem Brustbein auf, danach wird der Husten produktiv.

Der Auswurf ist von grau-gelber Farbe, die Schmerzen beim Husten verschwinden. Husten und Auswurf können 2 bis 3 Wochen anhalten. Eine kausale Therapie der Virusinfektion ist nicht möglich, eine bakterielle Superinfektion ist mit Antibiotika behandelbar.

Besteht im Verlauf der akuten Bronchitis der Verdacht auf eine bakterielle Superinfektiön, so ist in jedem Fall eine antibiotische Therapie mit Cephalosporinen der 2. und 3. Generation oder Makrolid-Antibiotika der neueren Generation angezeigt.

Aufgrund der primär viralen Genese des akuten Krankheitsbildes wird jedoch der Stellenwert der Antibiotikatherapie als sofortige Therapie der akuten Bronchitis sehr kontrovers diskutiert, insbesondere, wenn man die fortschreitende Resistenzbildung der Bakterien sowie ökonomische Gesichtspunkte hierbei bedenkt. Anhand von randomisierten Placebo-kontrollierten Studien mit Antibiotika in der Indikation "akute Bronchitis" konnte dieser Stellenwert der Antibiotika bisher nicht belegt werden.

Trotz dieser Kontroverse werden in der Praxis schätzungsweise bei mehr als 65% der Patienten mit der Diagnose akute Bronchitis Antibiotika verschrieben.

Unter Praxisbedingungen erfolgt jedoch häufig keine exakte Diagnosestellung, so daß Antibiotika ohne therapeutische Notwendigkeit eingesetzt werden. Der unkritische Einsatz von Antibiotika hat zu einer Zunahme der Resistenz von immer mehr Bakterien in Abhängigkeit von der Einsatzhäufigkeit geführt. Durch Antibiotika entsteht ein Selektionsdruck, der die Vermehrung resistenter Bakterienstämme begünstigt.

Beispielsweise treten zwischenzeitlich auch Staphylokokkenstämme mit verminderter Vancomycinresistenz auf, das ist deshalb dramatisch, als dieses Antibiotikum bisher als letztes Mittel gegen Bakterienstämme eingesetzt wurde, die gegen die üblicherweise eingesetzten Antibiotika resistent sind.

In dieser Situation besteht Bedarf an einer Behandlungsalternative.

Aufgabe der vorliegenden Erfindung ist es daher, eine pharmazeutische Zusammensetzung anzugeben, die den bisher verwendeten Antibiotika therapeutisch äquivalent und nebenwirkungsarm ist und keine Resistenzentwicklung begünstigt.

Diese Aufgabe wird durch die Merkmale der unabhängigen Ansprüche gelöst. Die abhängigen Ansprüche definieren vorteilhafte Ausführungsformen der Erfindung.

Überraschenderweise wurde gefunden, daß eine Kombination von Eukalyptusöl und Orangenöl in einem Gewichtsverhältnis zwischen 1:10 und 10;1, bevorzugt in einem Verhältnis von 2:1, in einer klinischen Prüfung nicht nur signifikant placeboüberlegen war, sondern auch einer Antibiotikatherapie äquivalent.

In einer doppelblinden, randomisierten, placebokontrollierten klinischen Studie bei Patienten mit akuter Bronchitis wurde die Wirksamkeit und Verträglichkeit einer 14tägigen Behandlung mit Eukalyptus-/Orangenöl untersucht. Als Parallelgruppe dienten Patienten, die über 14 Tage entsprechend mit Placebo oder Cefuroxim behandelt wurden.

Die Behandlungsgruppen umfaßten 170 Patienten mit Eukalyptus-/Orangenöl, 172 mit Placebo und 171 mit Cefuroxim.

Die Überlegenheit von Eukalyptus-/Orangenöl gegenüber Placebo konnte anhand der Studienabbrüche aufgrund einer Verschlimmerung der akuten Bronchitis nach 7±2 Behandlungstagen mit statistischer Signifikanz belegt werden. Prozentual betrug die Verschlimmerung unter Eukalyptus-/Orangenöl 7,1 %, unter Placebo hingegen 22,7 % und 7,6% bei den mit Cefuroxim behandelten Patienten.

Auch nach 14±2 Behandlungstagen kam es noch zu einer Verschlimmerung der akuten Bronchitis. In der Eukalyptus-/Orangenölgruppe betraf dies nur 1,8 % der Patienten, was sich wieder deutlich von der Placebo-Gruppe mit 36,6 % unterschied. In der Cefuroxim-Gruppe waren 16,4 % der Patienten betroffen, also ca. 10 mal mehr Patienten, als in der Eukalyptus-/Orangenölgruppe.

Als weiteres Indiz für den Behandlungserfolg zeigte sich nach 7±2 Behandlungstagen noch ein pathologischer Auskultationsbefund bei 27,1 % der mit Eukalyptus-/Orangenöl behandelten Patienten im Vergleich zu 48,3 % der Placebo-Patienten. In der Cefuroxim-Gruppe wiesen noch 26,3% eine pathologischen Auskultationsbefund auf. Nach 14±2 Tagen reduzierte sich der prozentuale Anteil an Patienten in der Eukalyptus-/Orangenöl-Gruppe auf 2,9 %, in der Placebo-Gruppe zeigten immer noch 14,5 % der Patienten pathologische Atemgeräusche. Bei mit Cefuroxim behandelten Patienten lag der prozentuale Anteil mit 7,6 % ebenfalls höher als in der Eukalyptus-/Orangenöl-Gruppe. Die beobachtete Hyperreagibilität des Bronchialsystems, die bei ca. 50 % aller Patienten bei einer ersten Visite beobachtet wurde, war bei mit Eukalyptus-/Orangenöl behandelten Patienten nach 7±2 Behandlungstagen deutlich niedriger als in der Placebo-Gruppe. Bezüglich der nächtlichen Hustenattacken, die die Nachtruhe beeinträchtigen, zeigte sich ab Tag 5 der Therapie in allen beiden Behandlungsgruppen im Vergleich zu Placebo eine deutliche Abnahme. Das Ausbleiben von Hustenattacken während des Tages wurde zwischen den 3 Venungruppen und Placebo erst nach einer Therapiedauer von 9 Tagen deutlich. Nach 14tägiger Behandlung war die Hälfte aller Eukalyptus-/Orangenöl-Patienten hustenfrei, bei Placebo jedoch lediglich 30 %. Die Wirksamkeit von Eukalyptus-/Orangenöl nach 7±2 bzw. 14±2 Tagen wurde von Ärzten und Patienten wesentlich besser beurteilt als Placebo und lag auch über der von Cefuroxim. Ebenso zeigte sich ab dem 4. Behandlungstag bereits ein deutlich besseres Allgemeinbefinden bei den mit Eukalyptus-/Orangenöl behandelten Patienten im Vergleich zur Placebo-Gruppe. In der Beurteilung des Allgemeinbefindens zeigten sich zwischen der Eukalyptus-/Orangenöl- und der Cefuroxim-Gruppe keine nennenswerten Unterschiede.

Bezüglich der Sicherheit und Verträglichkeit der Studienmedikation zeigt die körperliche Untersuchung sowie die Bestimmung der Labor- und Vitalparameter beim Abschlußbesuch im Vergleich zur ersten Visite keine relevanten Unterschiede zwischen den Behandlungsgruppen. Dies trifft auch auf Anzahl und Beurteilung der aufgetretenen unerwünschten Ereignisse zu und unterstreicht damit die gute Verträglichkeit von Eukalyptus-/Orangenöl.

Die Gabe von Eukalyptus-/Orangenöl bei akuter Bronchitis im Vergleich zu Placebo verhinderte bei 93% der Patienten eine Verschlimmerung des Krankheitsbildes und führte somit zu einer schnelleren Ausheilung. Eine sofortige Gabe von Antibiotika bei der akuten Bronchitis, wie oftmals praktiziert, war einer Behandlung mit Eukalyptus-/Orangenöl nachweislich nicht überlegen. Der frühzeitige Einsatz von Antibiotika in dieser Indikation sollte daher sowohl aus ökonomischen Gründen als auch wegen der Gefahr der Resistenzbildung genau überdacht werden.

Die obige klinische Studie zeigt, daß sich die Kombination von Eukalyptus-/Orangenöl hervorragend zur Behandlung von infektiösen Erkrankungen und Entzündungen der Atemwege eignet.

Die erfindungsgemäßen Eukalyptus-/Orangenöl-Kombinationen werden oral verabreicht. Erfindungsgemäß wird Eukalyptus-/Orangenöl in Form von Weich- oder Hartgelatinekapseln oder in flüssigen Darreichungsformen eingesetzt. Das Öl kann in reiner Form oder aber in Kombination mit anderen biologisch aktiven Stoffen, wie z.B. Menthol und üblichen Hilfsstoffen verwendet werden. Darüber hinaus kann es mit üblichen Wirkstoffen zur Behandlung einer vorliegenden Grunderkrankung kombiniert werden, z.B. mit Nitraten bei der Behandlung von cardialen Erkrankungen.

Bevorzugte Hilfsstoffe sind Lösungsvermittler. Als solche können organische Lösungsmittel, vorzugsweise Ethanol, Tenside, vorzugsweise Natrium-Dodecylsulfat, oder Natriumdesoxycholat, Kohlenhydrate, vorzugsweise Glucose oder Dextrose oder Lipide, vorzugsweise Phosphatidylcholin, sowie Mischungen dieser Stoffe verwendet werden. Die Lösungsvermittler werden üblicherweise in Mengen von 0,1 bis 20 Gew.-%, vorzugsweise 0,2 bis 15 Gew.-%, bezogen auf die Menge an Eukalyptus-/Orangenöl eingesetzt.

Im Fall von z.B. Hart- oder Weichgelatinekapseln liegt der Eukalyptus-/Orangenöl-Gehalt einer Kapsel vorzugsweise im Bereich von 1 bis 80 Gew.-%, besonders bevorzugt im Bereich von 16 bis 40 Gew.-% und ganz besonders bevorzugt im Bereich von 20 bis 35 Gew.-% . Eine Kapsel enthält vorzugsweise 40 bis 350 mg, besonders bevorzugt 100 bis 300 mg Eukalyptus-/Orangenöl. Die Tagesdosis liegt bei 0,01 bis 0,2 g Eukalyptus-/Orangenöl pro kg Körpergewicht.

Im Fall von flüssigen Darreichungsformen liegt der Eukalyptus/Orangenöl-Gehalt im Bereich von 0,1-10, bevorzugt 0,3-7, besonders bevorzugt 0,5-3 Gew.-%.

Das erfindungsgemäß verwendete Orangenöl enthält vorzugsweise überwiegend Limonen, besonders bevorzugt (+)-Limonen, d.h. mindestens 90 Gew.-%, vorzugsweise mindestens 93 Gew.-% und ganz besonders bevorzugt zwischen 93 und 98 Gew.-% der gesamten Orangenölmenge. Das Eukalyptusöl wird vorzugsweise in Form eines rektifizierten ätherischen Öls eingesetzt, das vorzugsweise einen Gehalt an 1,8-Cineol von mehr als 70 % sowie einen Gehalt an α-Pinen, vorzugsweise (+)-α-Pinen, aufweist.

Das Öl wird vorzugsweise als Lösung in einem Neutralöl oder einem natürlichen fetten Öl eingesetzt. Als natürliche Öle eignen sich Kokosnußöl, Rapsöl und Maisöl. Bevorzugte Neutralöle sind mittelkettige (C6- bis C12-) Triglyceride, insbesondere Capryl-Caprinsäure-Triglyceride, wie Miglyol 810 (Molgewicht ca. 520; gaschromato graphische Zusammensetzung: C6- max. 2 %; C8 65-75 %; C10 25-35 %; C12 max. 2 %) und Miglyol 812 (Molgewicht ca. 520; gaschromatographische Zusammensetzung: C6- max. 3 %; C8 50-65 %; C10 30-45 %; C12 max. 5 %).

Die Konzentration von Eukalyptus-/Orangenöl in einer oral einzunehmenden Lösung liegt vorzugsweise im Bereich von 5 bis 30 g/l, besonders bevorzugt im Bereich von 10 bis 20 g/l und ganz besonders bevorzugt im Bereich von 12 bis 18 g/l.

Bevorzugte flüssige Darreichungsformen sind Säfte und Emulsionen, die gemäß folgenden Beispielen 1 und 2 zusammengesetzt sein können:

### Beispiel 1: Eukalyptus- und Orangenöl enthaltender Saft

| Bestandteil | Gehalt [Gew.-%] |
|---|---|
| Macrogolglycerolhydroxystearat | 6 - 8 |
| Eukalyptus-/Orangenöl | 0,5 - 3,0 |
| Xylit | 18 - 22 |
| Carrageenan | 0,4 - 0,6 |
| tri-Natriumcitrat Dihydrat | 0,05 - 0,1 |
| Kaliumsorbat | 0,1 - 0,2 |
| Macrogol 1500 | 8 - 10 |
| Honigaroma | 0,1 - 0,2 |
| Zitronensäure | 0,1 - 0,2 |
| Aqua purificata | ad 100 % |

### Beispiel 2: Eukalyptus- und Orangenöl enthaltende Emulsion

| Bestandteil | Gehalt [Gew.-%] |
|---|---|
| Macrogolglycerolhydroxystearat | 6-8 |
| Eukalyptus-/Orangenöl | 0,5 - 3,0 |
| Miglyol 812 | 50 - 60 |
| Xanthan Gum | 8 - 12 |
| Zitronenaroma | 0,05 - 0,2 |
| Kaliumsorbat | 0,1 - 0,2 |
| Saccharin-Natrium | 0,04 - 0,08 |
| Zitronensäure | 1,5 - 2,5 |
| Aqua purificata | ad 100 % |

### Beispiel 3

Da Eukalyptusöl für sein toxikologisches Potential bekannt ist, wurde in einem in vitro-Versuch die Verträglichkeit von Eukalyptusöl und der erfindungsgemäßen Kombination aus Eukalyptus- und Orangenöl untersucht.

Hierzu wurden Kulturen von L-929-Zellen mit variablen Mengen (0,1 - 0,006 Vol.-%) an Eukalyptusöl allein und der erfindungsgemäßen Kombination aus Eukalyptus- und Orangenöl versetzt und 48 Stunden stehengelassen. Nach Ablauf dieses Zeitraums wurden die Anzahlen an lebenden Zellen in den mit den Ölen versetzten Kulturen bestimmt. Die erhaltenen Ergebnisse sind in den Tabellen 1 und 2 zusammengefaßt und der Zahl an lebenden Zellen in unbehandelten Kulturen ("Zellkontrolle") gegenübergestellt.

**Tab. 1:**

| Eukalyptusöl | | | | | |
|---|---|---|---|---|---|
| Konzentration | 0,0125% | 0,0100% | 0,0083% | 0,0071% | 0,0063% |
| | | | | | |
| Zellen x 10⁵/ml | 1,025 | 1,108 | 1,848 | 1,267 | 2,388 |
| | 0,775 | 0,591 | 1,601 | 1,128 | 1,450 |
| | 0,906 | 0,516 | 1,208 | 1,317 | 1,075 |
| | 1,016 | 0,503 | 1,272 | 0,757 | 1,061 |
| | | | | | |
| Mittelwerte | 0,931 | 0,680 | 1,482 | 1,117 | 1,494 |
| | | | | | |
| Standardabweichungen | ±0,117 | ±0,288 | ±0,298 | ±0,253 | ±0,623 |
| | | | | | |
| Angaben in % bezogen auf Zellkontrolle 2,38 x 10⁵/ml | 37,92% | 27,69% | 60,41% | 45,54 | 60,87% |

**Tab. 2:**

| Eukalyptus-/Orangenöl-Mischung | | | | | |
|---|---|---|---|---|---|
| Konzentration | 0,1000% | 0,0500% | 0,0250% | 0,0167% | 0,0125% |
| | | | | | |
| Zellen x 10⁵/ml | 1,337 | 2,410 | 3,597 | 3,017 | 5,345 |
| | 1,076 | 1,952 | 3,912 | 2,988 | 3,687 |
| | 1,431 | 2,483 | 3,562 | 3,941 | 4,077 |
| | 1,417 | 2,333 | 4,265 | 3,516 | 3,966 |
| | | | | | |
| Mittelwerte | 1,315 | 2,295 | 3,834 | 3,366 | 4,269 |
| | | | | | |
| Standardabweichungen | ±0,165 | ±0,236 | ±0,328 | ±0,454 | ±0,736 |
| | | | | | |
| Angaben in % .bezogen auf Zellkontrolle 4,33 x 10⁵/ml | 30,38% | 53,01% | 88,57% | 77,75% | 98,61% |

Während das Eukalyptusöl nur bis zu 0,008 % (v/v) vertragen wurde [Tab. 1], waren für die Kombination beider Öle bis 0,02 % (v/v) ohne zytotoxische Auswirkungen verträglich [Tab. 2]. Dies drückt sich in einer höheren Zahl an lebenden Zellen aus. Vergleicht man die entsprechenden Konzentrationen (0,0125%), so überleben 37,92 % unter Eukalyptusöl und 98,6% unter der Kombination. Diese Ergebnisse unterstützen die beobachtete gute klinische Verträglichkeit.

## Patentansprüche

1. Pharmazeutische Zusammensetzung für die orale Verabreichung in Form einer Hart- oder Weichgelatinekapsel umfassend Eukalyptusöl und Orangenöl.

2. Pharmazeutische Zusammensetzung nach Anspruch 1, wobei das Gewichtsverhältnis von Eukalyptusöl zu Orangenöl zwischen 1:10 und 10:1 beträgt.

3. Pharmazeutische Zusammensetzung nach Anspruch 1 oder 2, wobei das Gewichtsverhältnis von Eukalyptusöl zu Orangenöl 2:1 beträgt.

4. Pharmazeutische Zusammensetzung nach einem der Ansprüche 1-3, wobei der Gehalt an Eukalyptusöl und Orangenöl 1-80 Gew.-%, bevorzugt 10-75 Gew.-%, besonders bevorzugt 40-70 Gew.-% beträgt.

5. Pharmazeutische Zusammensetzung nach einem der Ansprüche 1-4, wobei die Zusammensetzung des weiteren pharmazeutisch akzeptable Hilfsstoffe wie Lösungsvermittler und/oder Füllstoffe umfasst.

6. Pharmazeutische Zusammensetzung für die orale Verabreichung in Form einer Flüssigkeit umfassend Eukalyptusöl und Orangenöl.

7. Pharmazeutische Zusammensetzung nach Anspruch 6, wobei das Gewichtsverhältnis von Eukalyptusöl zu Orangenöl zwischen 1:10 und 10:1 beträgt.

8. Pharmazeutische Zusammensetzung gemäß Anspruch 6 oder 7, wobei das Gewichtsverhältnis von Eukalyptusöl zu Orangenöl 2:1 beträgt.

9. Pharmazeutische Zusammensetzung gemäß einem der Ansprüche 6-8, wobei der Gehalt an Eukalyptusöl und Orangenöl 0,1-10 Gew.-%, bevorzugt 0,3-7 Gew.-%, besonders bevorzugt 0,5-3 Gew.-% beträgt.

10. Pharmazeutische Zusammensetzung gemäß einem der Ansprüche 6-9, wobei die Zusammensetzung des weiteren pharmazeutisch akzeptable Hilfsstoffe umfasst.

11. Pharmazeutische Zusammensetzung gemäß einem der Ansprüche 6-10, wobei die Zusammensetzung ein Saft oder eine Emulsion ist.

12. Verwendung einer pharmazeutischen Zusammensetzung nach einem der Ansprüche 1-11 für die Herstellung eines Arzneimittels für die Behandlung infektiöser Erkrankungen und Entzündungen der Atemwege.

## Claims

1. A pharmaceutical composition for oral administration in the form of a hard or soft gelatine capsule comprising Eucalyptus oil and orange oil.

2. The pharmaceutical composition according to claim 1, wherein the ratio by weight of Eucalyptus oil to orange oil is between 1:10 and 10:1.

3. The pharmaceutical composition according to claim 1 or 2, wherein the ratio by weight of Eucalyptus oil to orange oil is 2:1.

4. The pharmaceutical composition according to any one of the claims 1 to 3, wherein the content of Eucalyptus oil and orange oil is 1 to 80 percent by weight, preferably 10 to 75 percent by weight and particularly 40 to 70 percent by weight.

5. The pharmaceutical composition according to any one of the claims 1 to 4, wherein the composition furthermore comprises pharmaceutically acceptable inert ingredients such as solubilizers and/or fillers.

6. A pharmaceutical composition for oral administration in the form of a liquid comprising Eucalyptus oil and orange oil.

7. The pharmaceutical composition according to claim 6, wherein the ratio by weight of Eucalyptus oil to orange oil is between 1:10 and 10:1.

8. The pharmaceutical composition according to claim 6 or 7, wherein the ratio by weight of Eucalyptus oil to orange oil is 2:1.

9. The pharmaceutical composition according to any one of the claims 6 to 8, wherein the content of Eucalyptus oil and orange oil is 0.1 to 10 percent by weight, preferably 0.3 to 7 percent by weight and especially 0.5 to 3 percent by weight.

10. The pharmaceutical composition according to any one of the claims 6 to 9, wherein the composition furthermore comprises pharmaceutically acceptable inert ingredients.

11. The pharmaceutical composition according to any one of the claims 6 to 10, wherein the composition is a syrup or an emulsion.

12. The use of a pharmaceutical composition according to any one of the claims 1 to 11 for the manufacture of a medicament for the treatment of infectious diseases and inflammations of the respiratory tract.

## Revendications

1. Composition pharmaceutique pour administration par voie orale sous la forme d'une gélule dure ou molle, comprenant de l'huile d'eucalyptus et de l'huile d'orange.

2. Composition pharmaceutique selon la revendication 1, où le rapport du poids d'huile d'eucalyptus sur le poids d'huile d'orange est compris entre 1/10 et 10/1.

3. Composition pharmaceutique selon la revendication 1 ou la revendication 2, où le rapport du poids d'huile d'eucalyptus sur le poids d'huile d'orange est de 2/1.

4. Composition pharmaceutique selon l'une des revendications 1 à 3, où la teneur en huile d'eucalyptus et en huile d'orange est comprise entre 1 et 80 % en poids, de préférence entre 10 et 75 % en poids, et de préférence encore entre 40 et 70 % en poids.

5. Composition pharmaceutique selon l'une des revendications 1 à 4, où la composition comprend en outre des adjuvants pharmaceutiquement acceptables tels que des agents de solubilisation et/ou des agents de charge.

6. Composition pharmaceutique pour administration par voie orale sous la forme d'un liquide, comprenant de l'huile d'eucalyptus et de l'huile d'orange.

7. Composition pharmaceutique selon la revendication 6, où le rapport du poids d'huile d'eucalyptus sur le poids d'huile d'orange est compris entre 1/10 et 10/1.

8. Composition pharmaceutique selon la revendication 6 ou la revendication 7, où le rapport du poids d'huile d'eucalyptus sur le poids d'huile d'orange est de 2/1.

9. Composition pharmaceutique selon l'une des revendications 6 à 8, où la teneur en huile d'eucalyptus et en huile d'orange est comprise entre 0,1 et 10 % en poids, de préférence entre 0,3 et 7 % en poids, et de préférence encore entre 0,5 et 3 % en poids.

10. Composition pharmaceutique selon l'une des revendications 6 à 9, où la composition comprend en outre des adjuvants pharmaceutiquement acceptables.

11. Composition pharmaceutique selon l'une des revendications 6 à 10, où la composition est un sirop ou une émulsion.

12. Utilisation d'une composition pharmaceutique selon l'une des revendications 1 à 11 pour la préparation d'un médicament destiné au traitement d'inflammations et de maladies infectieuses des voies respiratoires.
